# EUROPÄISCHE PATENTANMELDUNG

(11) **EP 2 333 505 A1**
(43) Veröffentlichungstag der Anmeldung: **15.06.2011**
(21) Anmeldenummer: 09178356.3
(22) Anmeldetag: 08.12.2009
(51) Int. Cl.: G01K 3/00, C12Q 1/34

(54) **Zusammensetzung und verfahren zur Temperaturüberwachung von kühl- und Tiefkühlerzeugnissen**

(71) Anmelder: BASF SE, 67056 Ludwigshafen (DE)
(72) Erfinder: Piatesi, Andrea, 68165, Mannheim (DE); Müller, Bonnie Christine, 68623, Lampertheim (DE); Fingerle, Andrea, 67271, Battenberg (DE)
(74) Vertreter: Popp, Andreas

(57) **Zusammenfassung**

Die Erfindung betrifft einen Indikator zur Temperaturüberwachung eines gekühlten oder tiefgekühlten Erzeugnisses, umfassend ein Enzym, das die Umsetzung eines Polysaccharids zu einem Monosaccharid katalysiert, ein Enzym, das die Oxidation des gebildeten Monosaccharids katalysiert, und ein Gemisch, das zum Nachweis der Enzymaktivitäten geeignet ist und das einen Farbindikator enthält. Weiterhin betrifft die Erfindung ein Verfahren zum Herstellen des Indikators, Verfahren zur Temperaturüberwachung, sowie Verpackungen, die mit dem erfindungsgemäßen Indikator versehen sind.

## Beschreibung

### GEBIET DER ERFINDUNG

Die vorliegende Erfindung betrifft das Gebiet der Tiefkühlung von temperatursensiblen Gegenständen, insbesondere Nahrungsmittel.

### HINTERGRUND DER ERFINDUNG

Bei der Tiefkühllagerung von Erzeugnissen, z.B. Nahrungsmitteln, muss, wenn eine längere Haltbarkeit gewährleistet sein soll, eine Lagertemperatur von mindestens -16°C eingehalten werden. Wird dagegen eine Temperatur von -10°C bis -15°C überschritten, so ist die Haltbarkeit vieler Erzeugnisse nicht mehr gewährleistet; das Tiefkühlgut gilt als angetaut, obwohl es äußerlich noch gefroren erscheint.

Normalerweise sind Tiefkühlgeräte mit eingebauten Thermometern ausgerüstet, welche die Temperatur an einer bestimmten Stelle des Kühlraumes anzeigen, oder es wird ein gebräuchliches Thermometer auf oder neben das Kühlgut gelegt und zum Ablesen aus dem Kühlgerät herausgenommen. In diesen Fällen besteht keine Gewähr, dass die Temperatur des Kühlgutes genau gemessen wird. Ausserdem kann durch Stapelung und Packung des Kühlgutes die Konvektion der Luft im Kühlraum behindert werden, so dass selbst bei einwandfreier Funktion des Gerätes an einigen Stellen des Kühlraumes die für die Haltbarkeit der Erzeugnisse erforderlichen Mindesttemperaturen überschritten werden können. Für den Benutzer des Tiefkühlgutes ist das Überschreiten der Haltbarkeitstemperatur nicht zu erkennen, so dass er dem Verderb der Erzeugnisse nicht durch rechtzeitige Entnahme und alsbaldigen Verbrauch begegnen kann. Die Gefahr, dass tiefgefrorene Erzeugnisse unbemerkt angetaut werden, ist beispielsweise besonders gross, wenn eine grössere Menge Kühlgut warm in das Tiefkühlgerät eingebracht wird. Daher ist es wünschenswert, einen Temperatur-Indikator zur Verfügung zu haben, der für jede Nahrungsmittelverpackung oder eine Gruppe von Packungen anzeigt, ob sie während ihrer gesamten Lagerzeit hinreichend gekühlt waren.

Durch im Stand der Technik bekannte Temperaturindikatoren können Unterbrechungen innerhalb einer Kühl/Tiefkühlkette nachgewiesen werden. Das Mess-Prinzip basiert meist auf physikalischen, chemischen, mikrobiologischen oder enzymatischen Prozessen. Je nach System erhält man unterschiedliche Reaktionen auf eine Unterbrechung der Kühl/Tiefkühlkette wie z. B. Farbveränderung, Farbentwicklung oder eine Wanderung eines Farbstoffes entlang einer Skala.

Dem Fachmann als "Partial-History" Integratoren bekannte Indikatoren zeigen erst eine Reaktion, wenn eine definierte Schwellentemperatur überschritten wurde. "Full-History" Integratoren reagieren kontinuierlich und zeigen, ab dem Zeitpunkt der Aktivierung, die gesamte Temperatur-Zeit-Historie an. Dieses System funktioniert unabhängig von einer Schwellentemperatur. (J.H. Wells, R.P. Singh (1987) - A graphical Interpretation of time-temperature related quality changes in frozen food. J. Food Sci. 52, 436 - 439). Nach Taoukis und Labuza (Proc. Of the International Symposium Quimper Froid, 1997, 291-297) werden Indikatoren in drei Gruppen eingeteilt: (i) "Critical Temperature Indicators" (CTI) - Bei Überschreitung einer definierten Temperatur kommt es zu einer irreversiblen Veränderung des Labels; (ii) "Critical-Time-Temperature lndicators" (CTTI) -zeigen eine kumulative Temperatur- Zeit- Historie über einer definierten Schwellentemperatur an; (iii) "Time-Temperature-Indicators" (TTI) - Werden aktiviert und zeigen dann die gesamte Temperatur-Zeit- Historie an.

An solche Frischhalteindikatoren, dem Fachmann auch als "Labels" bekannt, werden verschiedene Ansprüche gestellt. Wichtig ist vor allem die Stabilität des Indikators. Darunter versteht man eine stabile Formulierung vor der Aktivierung und ein anschließendes lineares Verhalten. Der Indikator sollte durch äußere Faktoren, wie z.B. UV-Licht und Feuchtigkeit, nicht beeinflussbar sein. Für den Lebensmittelproduzenten muss das Label vor allem günstig und einfach anwendbar sein. Ein einfaches Aufbringen des Labels auf die Verpackung ist Voraussetzung, um ein neues System möglichst einfach in den Verpackungsprozess zu integrieren und um zusätzliche Kosten, wie den Kauf einer neuen teuren Etikettiermaschine, zu vermeiden. Für den Verbraucher muss das Label leicht verständlich und der detektierte Frischegrad auf einen Blick erfassbar sein.

Temperatur- oder Temperatur-Zeit-Indikatoren sind aus dem Stand der Technik seit längerem bekannt. Das System "3M MonitorMark" beispielsweise beruht auf der Diffusion eines blau gefärbten Esters entlang einer Skala. Die Überschreitung einer definierten Schwellentemperatur führt zu einem irreversiblen Schmelzen des Esters, wobei die gesamte Zeit-Temperatur-Historie bestimmt werden kann. (T.P. Labuza (2000) The search for shelf life-An update on continued efforts in understanding practical strategies for determining and testing the shelf life of food products. Food Testing Analysis,)

Der kommerziell erhältliche "VITSAB"-lndikator beruht auf einer enzymatischen Hydrolyse von Tricaproin durch eine Pankreas-Lipase. Das Enzym und die Substratlösung mit pH-Indikator sind in einem Zweikammersystem voneinander getrennt. Die Aktivierung des Labels erfolgt durch einen Druck auf das Label, wobei die dünne Barriere zwischen Enzym und Substratlösung gebrochen wird. Je wärmer die Außentemperatur, desto schneller erfolgt die Vermischung beider Lösungen und desto schneller ist die enzymatische Hydrolyse. Die Entstehung von Caprinsäure führt zu einer pH- Senkung und so zu einem Farbwechsel von grün nach gelb. Nachteil dieses Systems ist allerdings die Inaktivierung des Enzyms bei einer Temperatur von über 40°C, was zu einer Fehlfunktion des Indikators führen kann. (T. Labuza, Bin Fu, Use of Time/Temperature Integrators, Predictive Microbiology, and Related Technologies for Assessing in the extent and impact of temperature abuse on meat an poultry products, Journal of Food safety 15 (1995); 201-227; S. Tsoka, P.S. Taoukis, Time Temperature Integrators for chilled food shelf life monitoring using enzyme-substrate systems, Food Biotechnology 12 (1&2) (1998), 139-155)

Das "Fresh Check"-System beruht auf der Polymerisation eines farblosen Acetylen- oder Diacetylen-Monomers zu einem farbigen Polymer. Die Länge und somit die Farbintensität sind temperaturabhängig. Die Reaktion erfolgt in der Mitte des Labels, wobei der dunklere Kreis um die helle Fläche den Referenzring darstellt. Das System weist den Nachteil auf, dass die Labels ab dem Zeitpunkt ihrer Herstellung aktiv sind und bei -25°C gelagert werden müssen. (P.S. Taoukis, T.P. Labuza (1989) - Applicability of time- temperature indicators as shelf life monitors of food products. J. Food Sci., 54 (4), 783-788)

Das so genannte "OnVu-Label" basiert auf der Aktivierung eines Dinitrobenzylpyridin (DNBP)-Labels mit UV-Licht. Die Blaufärbung beruht auf einer lichtinduzierten Protonen-Transfer Reaktion, bei der das blaue Photoprodukt durch "thermisch aktives Tunneln" wieder langsam in den Grundzustand zurückkehrt und dabei wieder farblos wird. Nach Ablauf der für das Kühlgut berechneten Haltbarkeitsdauer und bei Kühlkettenabbrüchen ist eine (nahezu) vollständige Entfärbung des Etiketts erkennbar. Um eine wiederholte Aktivierung des Etiketts zu verhindern, muss der aktive Aufdruck mit einer UV-undurchlässigen Folie überklebt werden. (J. Kreyenschmidt (2003) - Modellierung des Frischeverlustes von Fleisch sowie des Entfärbeprozesses von Temperatur- Zeit- Integratoren zur Festlegung von Anforderungsprofilen für die produktbegleitende Temperaturüberwachung, Diss. Universität Bonn)

Wegen häufiger Presseberichte über Missbräuche bei der Lebensmittellagerung ist die Einhaltung der Tiefkühlkette wieder ein aktuelles Thema geworden. Die meisten kommerziell erhältlichen Temperatur-Indikator-Systeme haben den Nachteil, dass sie besondere Lagerungsbedingungen erfordern, um nicht frühzeitig aktiviert zu werden. Darüber hinaus soll die Visualisierung einer Temperaturänderung auf der Verpackung möglichst ohne weitere Schritte auf Seiten der Verbraucher durchgeführt werden können.

### ZUSAMMENFASSUNG DER ERFINDUNG

Demgemäß bestand die objektive technische Aufgabe der Erfindung darin, ein Verfahren bereit zu stellen, mittels dessen eine Unterbrechung in der Kühlkette möglichst einfach, zuverlässig und kostengünstig nachgewiesen werden kann.

Die Erfinder haben einen enzymbasierten CTI entwickelt, der in Form einer Flüssigkeit (auch als "smart ink" bezeichnet), auf papierbasierten Verpackungen - kurz vor dem Einfrieren - gedruckt werden kann. Dabei sollen grobe Verstöße innerhalb einer Tiefkühlkette aufgezeigt werden.

Die Aufgabe wurde demnach durch den Gegenstand der Erfindung gelöst, betreffend einen Indikator zur Temperaturüberwachung eines gekühlten oder tiefgekühlten Erzeugnisses, umfassend:
a. mindestens ein Enzym, das die Umsetzung eines Polysaccharids zu einem Monosaccharid katalysiert,
b. mindestens ein Enzym, das die Oxidation des mittels a) gebildeten Monosaccharids katalysiert,
   und
c. ein Gemisch, das zum Nachweis der Enzymaktivität aus a) und/oder b) geeignet ist und das einen Farbindikator enthält,
dadurch gekennzeichnet, dass die Enzymaktivität erst nach Aufbringen auf eine Polysaccharid-Trägermatrix in Anwesenheit von Wasser und in einem Temperaturbereich, bei dem das Erzeugnis aufgetaut vorliegt, nachweisbar ist.

In einer bevorzugten Ausführungsform ist der Indikator auf einem Polysaccarid, vorzugsweise Cellulose, aufgebracht und getrocknet.

In weiteren bevorzugten Ausführungsformen umfasst der Farbindikator ein chromogenes Substrat für eine Peroxidase oder für eine Hydrolase, insbesondere eine Perhydrolase.

Ein weiterer Gegenstand betrifft ein Verfahren zur Herstellung eines erfindungsgemäßen Indikators.

Ein weiterer Gegenstand der Erfindung betrifft ein Verfahren zur Temperaturüberwachung eines gekühlten oder tiefgekühlten Erzeugnisses, umfassend das Aufbringen eines erfindungsgemäßen Indikators auf das Erzeugnis und das Kontrollieren der Enzymaktivität anhand des Farbindikators, wobei der positive Nachweis der Enzymaktivität indikativ für eine Unterbrechung der Kühlung oder Tiefkühlung des Erzeugnisses ist.

Vorzugsweise erfolgt das Aufbringen in einem Temperaturbereich, bei welchen das Enzym aus a) und/oder das Enzym aus b) und/oder das Gemisch aus c) inaktiv sind.

Ein weiterer Gegenstand der Erfindung betrifft eine Verpackung zur Aufbewahrung eines gekühlten oder tiefgekühlten Erzeugnisses, dadurch gekennzeichnet, dass die Verpackung mit wenigstens einem ortsfesten oder entfernbaren, erfindungsgemäßem Indikator versehen ist.

Vorzugsweise ist der Indikator wenigstens zwischen einer äußeren, sichtbaren und/oder UV- Licht durchlässigen, vorzugsweise Wasser-undurchlässigen, Schicht und/oder einer für den Indikator undurchlässigen, dem Erzeugnis zugewandten, inneren Schicht angeordnet.

Schließlich betrifft die Erfindung die Verwendung einer erfindungsgemäßen Verpackung zur Aufbewahrung von gekühlten oder tiefgekühlten Erzeugnissen.

### BESCHREIBUNG DER ZEICHNUNG

Figur 1
   Flussdiagramm der Enzymkaskade des erfindungsgemäßen Indikators
Figur 2
   Mikrotiterplatte (MTP) mit Versuchsaufbau der "Smart lnk" Formulierung; Reihe 1: nur Onozuka Zellulase; Reihe 2: nur beta-Glukosidase; Reihe 3: Onozuka Zellulase und beta-Glukosidase; Reihe 4: Glucose-Standard; Letzte Spalte: kein Enzym, Wasserkontrolle
Figur 3
   Versuchsaufbau Peroxidase-Label in Mikrotiterplatte enthaltend Zellstoff-Blättchen nach 30min (a) und 6 Stunden (b) Inkubation bei Raumtemperatur. Reihe 1: nur Onozuka Zellulase; Reihe 2: nur beta-Glukosidase; Reihe 3: Onozuka Zellulase und beta-Glukosidase; Letzte Spalte: kein Enzym, Wasserkontrolle
Figur 4
   Bestimmung der verschiedenen Enzymformulierungen auf Anwesenheit von Glucose.
Figur 5
   Versuchsaufbau Perhydrolase-Label in Mikrotiterplatte enthaltend Zellstoff-Blättchen nach 3 Stunden (a) und 6 Stunden (b) Inkubation bei Raumtemperatur. Reihe 1: 44 µg Phenolrot; Reihe 2: 88 µg Phenolrot; Letzte Spalte von a) und b): kein Enzym, Wasserkontrolle.

### DEFINITIONEN

Der Ausdruck "Indikator" bezeichnet eine Vorrichtung, einen Gegenstand, und/oder ein Material, welches sich in detektierbarer Weise in Abhängigkeit von der Temperatur, welcher er ausgesetzt ist, verändern kann. Die Veränderung kann physikalischer oder chemischer Natur oder beides sein, und lässt sich mittels menschlichem Sehvermögen, Geruchs-/Tastsinn und/oder unter Zuhilfenahme von elektrischen, chemischen oder strahlungsbasierten Untersuchungsgeräten nachweisen.

Der Ausdruck "Temperaturüberwachung" beschreibt die Überwachung der Kühl- oder Tiefkühlkette bei leicht verderblichen Erzeugnissen, beispielsweise Lebensmittel, Impfstoffen und Blut sowie bei anderen Anwendungen. Im Zusammenhang mit der vorliegenden Erfindung wird darunter insbesondere die Überwachung dessen verstanden, ob ein gekühltes, insbesondere ein tiefgekühltes Erzeugnis aufgetaut vorliegt oder vorgelegen hat, das heißt, auf eine Temperatur von mindestens minus 15°C, mindestens minus 5°C, bevorzugt mindestens 0°C, besonders bevorzugt mindestens 10°C, ganz besonders bevorzugt auf eine Temperatur von mindestens 15°C erwärmt wurde.

Der Ausdruck "gekühltes Erzeugnis" bezieht sich auf ein Erzeugnis, welches bei einer Temperatur von etwa minus 5°C bis plus 10°C, vorzugsweise von etwa minus 3°C bis etwa plus 8°C, besonders bevorzugt 0°C bis plus 5°C gelagert werden muss. Der Ausdruck "tiefgekühltes Erzeugnis" bezieht sich auf ein Erzeugnis, welches bei einer Temperatur von etwa minus 80°C bis minus 5°C, vorzugsweise minus 50°C bis minus 8°C, besonders bevorzugt minus 30°C bis minus 10°C, ganz besonders bevorzugt von etwa minus 25°C bis minus 15°C gelagert werden muss. Tiefgekühlt ist im Rahmen der vorliegenden Erfindung gleichbedeutend mit gefroren oder eingefroren.

Der Ausdruck "Polysaccharid" bzw. Vielfachzucker umfasst im Rahmen der vorliegenden Erfindung natürliche Polysaccharide, synthetische Polysaccharide, Polysaccharid-Derivate, modifizierte Polysaccharide, sowie entsprechende Mischungen davon. Eingeschlossen sind chemische Verbindungen, die aus einer Vielzahl (mehreren Hundert bis Tausende) Monosaccharid-Einheiten pro Molekül aufgebaut sind und aus geraden oder verzweigten Ketten bestehen können. Ihre Molekulargewichte bewegen sich üblicherweise im Bereich über 5000 und können bis Millionen Dalton erreichen. Üblicherweise handelt es sich bei Polysacchariden um natürlich vorkommende Polymere wie Glycogen, Stärke (Amylose und Amylopektin) und Stärkederivate, Cellulose, Hemicellulose, wie z.B. Xylan, Mannan, (Arabino-) Galactan, Dextran, Pektin, Guargummi, Scleroglucan, Gummi arabicum, Agar, Chitin, Callose, Xanthan, Johannisbrotkernmehl (Carob-Gummi), Chitosan, Algin, Carrageenan, Gellan, Welan, Rhamsan, Curdlan, Lignin, Tamarindengummi und Pullulan.

Wenn das Polysaccharid Stärke ist, kann das Stärke-basierende Material, das für die Erfindung geeignet ist, jede beliebige Stärke sein. Solche Stärkesorten umfassen solche von jeder beliebigen Pflanzensorte einschließlich Mais, Kartoffel, Süßkartoffel, Mehl, Reis, Tapioka, Sago, Hirse. Umfasst sind ebenfalls Umwandlungsprodukte, deren Grundlage die zuvor genannten Stärken sind, beispielsweise Dextrine, die durch saure Hydrolyse oder Wärmeeinwirkung hergestellt werden, sowie derivatisierte oder kreuzvernetzte Stärken.

Der Ausdruck "Monosaccharid" umschreibt organisch-chemische Verbindungen mit mindestens drei Kohlenstoffatomen. Umfasst sind Glycerinaldehyd; Erythrose; Threose; Ribose; Arabinose; Xylose; Lyxose; Allose; Altrose; Glucose; Mannose; Gulose; Idose, Galactose; Talose. Für die vorliegende Erfindung ist es besonders bevorzugt, wenn das Monosaccharid Glucose ist.

Der Ausdruck "Oxidation" bezeichnet eine chemische Reaktion, bei der ein zu oxidierender Stoff (Elektronendonator) Elektronen abgibt. Ein anderer Stoff (Oxidationsmittel) nimmt die Elektronen auf (Elektronenakzeptor) und wird dadurch reduziert. Beide Reaktionen zusammen werden als Teilreaktionen einer Redoxreaktion verstanden. Enzyme, die eine Oxidations-/Reduktionsreaktion katalysieren, kennt der Fachmann unter der Bezeichnung "Oxidasen", wobei molekularer Sauerstoff (O₂) als Elektronenakzeptor fungiert, welcher zu Wasser (H₂O) oder Wasserstoffperoxid (H₂O₂) reduziert wird. Das entstehende Wasserstoffperoxid dient als Komponente des Gemisches c. des erfindungsgemäßen Indikators im Rahmen der vorliegenden Erfindung als weiteres Oxidationsmittel, welches in Verbindung mit dem "Farbindikator" zum Nachweis der Enzymaktivität geeignet ist.

Der Ausdruck "Farbindikator" bezeichnet einen Stoff, mit dessen Hilfe sich der Verlauf eines physikalischen, chemischen, mikrobiologischen oder vorzugsweise enzymatischen Prozesses verfolgen oder der Zustand eines physikalischen, chemischen, mikrobiologischen oder vorzugsweise enzymatischen Systems charakterisieren lässt, wobei eine Zustandsänderung durch einen ein- oder zweifarbigen Farbumschlag signalisiert wird. Je nach Verwendungszweck und Indikationsprinzip werden im Stand der Technik unterschieden: pH-Indikatoren (Säure-Base-Indikatoren), Redoxindikatoren, Metallindikatoren, Adsorptionsindikatoren, Fluoreszenzindikatoren, Chemilumineszensindikatoren. Die Farbänderung eines Indikators ist auf das Auftreten strukturell unterschiedlicher Formen (z.B. durch Protolyse oder Deprotolyse infolge einer pH-Änderung) oder die Reaktion des Indikators mit einer der Komponenten des Reaktionssystems unter Bildung einer andersfarbigen Verbindung zurückzuführen. In Abhängigkeit vom pH-Wert oder des Redoxzustandes ändert sich die Lichtabsorption des Farbindikators, was durch einen Farbumschlag im sichtbaren Spektralbereich nachweisbar ist. Derartige Substanzen sind dem Fach auch als "chromogene" Substanzen, d.h. Substanzen die zu einem Farbumschlag befähigt sind, geläufig. Als Umschlagbereich bezeichnet man den pH-Bereich, in dem visuell der Farbumschlag des Indikators verfolgt werden kann und erstreckt sich ungefähr über einen Intervall von 1-2 pH Einheiten. Eine Auswahl von gängigen Farbindikatoren, die dem Fachmann bekannt sind, sind in der folgenden Tabelle 1 zusammengefasst.

| CAS-Reg-No | Farbindikator | pK Hln | Indikatorsäure | Umschlagsbereich (pH) | Indikatorbase |
|---|---|---|---|---|---|
| [554-73-4] | Tropäolin | | violettrot | 1,2-3,2 | gelborange |
| [76-61-9] | Thymolblau (1. Stufe) | 1,5 | rot | 1,2-2,8 | gelb |
| [117-92-0] | Chinaldinrot | | farblos | 1,4-3,2 | rosa |
| [547-58-0] | Methylorange | 4,2 | orange | 3,1-4,4 | gelb |
| [573-58-0] | Kongorot | 3,7 | blau | 3,0-5,2 | rot |
| [76-60-8] | Bromkresolgrün | 4,7 | gelb | 3,8-5,4 | blau |
| [493-52-7] | Methylrot | 5,1 | rot | 4,2-6,3 | gelb |
| [1393-92-6] | Lackmus | | rot | 5,0-8,0 | blau |
| [76-59-5] | Bromthymolblau | 7,0 | gelb | 6,0-7,7 | blau |
| [143-74-8] | Phenolrot | 7,9 | gelb | 6,8-8,4 | rot |
| [76-61-9] | Thymolblau (2. Stufe) | 8,9 | gelb | 8,0-9,6 | blau |
| [77-09-8] | Phenolphthalein | 9,4 | farblos | 8,2-10,0 | rot |
| [125-20-2] | Thymolphthalein | 9,9 | farblos | 9,3-10,5 | blau |
| [584-42-9] | Alizaringelb GG | | hellgelb | 10,0-12,1 | bräunlich-gelb |
| [84540-31-8] | Epsilonblau | | orange | 12,0-13,0 | violett |

Darüber hinaus sind dem Fachmann "chromogene Substrate" oder Indikatorenzymsubstrate bekannt. Darunter werden chromogene Substanzen verstanden, welche an besonderen enzymatischen Reaktionen entweder als Elektronendonor oder als Elektronenakzeptor teilnehmen können und welche im Verlauf dieser Reaktion ihre Farbe ändern. Zum Beispiel katalysiert das Enzym Peroxidase die Umwandlung von Wasserstoffperoxid zu Wasser. Sofern das Donormolekül ein chromogenes Substrat ist, bewirkt dessen Oxidation einen nachweislichen Farbumschlag. Beispiele solcher Substrate sind für β-Galaktosidase die Galaktoside von Resorufin, Chlorphenolat oder Nitrophenol. Beispiele für Peroxidasesubstrate sind Resorufin und Triarylimidazole. Beispielsweise ist 3, 3', 5, 5'-tetramethylbenzidine (TMB) farblos im reduzierten Zustand, aber blau im oxidierten Zustand bzw. gelb im Diaminzustand. Im Falle von Reaktionen unter Einfluss der alkalischen Phosphatase wird das farblose p- Nitrophenylphosphate (pNPP) in seine farblose p-Nitrophenoxid-Form (benzenoide Form) umgewandelt, bei alkalischem pH von der benzenoiden in die quininoide Form, welche gelb ist. Für die vorliegende Erfindung geeignete chromogene Substrate umfassen, ohne darauf beschränkt zu sein 4-Chloro-1-naphtol (4-CN), ortho-phenylenediamine (OPD), 2, 2'-azinobis- (3- ethyl-benzothiazoline-6-sulfonic acid) (ABTS), diaminobenzidine (DAB), 3,3'dimethyloxybenzidine (ortho- dianisidine oder ODN), 5-aminosalicylic acid (5AS), und 3, 3', 5, 5'-tetraalkylbenzidine wie zum Beispiel 3, 3', 5, 5'- tetramethylbenzidine (TMB). Andere chromogene Substrate umfassen verschiedene fluoreszente und chemilumineszente Verbindungen. Eine Liste chromogener Substrate mit den möglichen Farbumschlägen ist in Tabelle 2 dargestellt.

**Tabelle 2:**

| Chromogenes Substrat | reduziert | oxidiert | Absorptions-max (nm) |
|---|---|---|---|
| OPD | farblos | gelb | 450 |
| ABTS | farblos | blau-grün | 405-410 |
| DAB | farblos | braunes Präzpitat | |
| OND | farblos | gelb-orange | 460 |
| TMB | farblos | blau | 650 |
| 5AS | farblos | braun | 450 |
| o-Toluidin | farblos | rot | |

### AUSFÜHRLICHE BESCHREIBUNG

Das erfindungsgemäße Indikatorsystem eignet sich für grobe Verstöße innerhalb einer Tiefkühlkette und zeigt keine Zeit- und Temperatur Historie an. Das System beruht auf einer aufeinander abgestimmten Enzymkaskade, die erst bei Raumtemperatur eine Reaktion zeigt. So werden kurze Unterbrechungen der Tiefkühlkette, die z.B. beim Verladen der Ware entstehen können, nicht detektiert.

Ein erster Gegenstand der Erfindung betrifft demnach einen Indikator zur Temperaturüberwachung eines gekühlten oder tiefgekühlten Erzeugnisses, umfassend die Komponenten:
a. mindestens ein Enzym, das die Umsetzung eines Polysaccharids zu einem Monosaccharid katalysiert,
b. mindestens ein Enzym, das die Oxidation des mittels a) gebildeten Monosaccharids katalysiert,
   und
c. ein Gemisch, das zum Nachweis der Enzymaktivität aus a) und/oder b) geeignet ist und das einen Farbindikator enthält,
dadurch gekennzeichnet, dass die Enzymaktivität erst nach Aufbringen auf eine Polysaccharid-Trägermatrix in Anwesenheit von Wasser und in einem Temperaturbereich, bei dem das Erzeugnis aufgetaut vorliegt, nachweisbar ist.

Demgemäß liegt der erfindungsgemäße Indikator zunächst in einem inaktiven Zustand vor, bevor er auf das zu kühlende oder auf das zu gefrierende Erzeugnis aufgebracht wird. Eine Aktivierung des Indikators erfolgt nur dann, wenn folgende Bedingungen erfüllt sind:
(1) der Indikator wird mit einem für das Enzym (a) spezifischen Polysaccharid in Kontakt gebracht, und/oder
(2) der Indikator wird mit einem für das Enzym (b) spezifischen Monosaccharid in Kontakt gebracht; und
(3) der Indikator wird in ein wässriges Medium und in einen Temperaturbereich, bei denen die Enzyme in (a), (b) und ggf (c) eine katalytische Aktivität aufweisen, überführt.

Voraussetzung für die Erfindung ist daher einerseits, dass die Komponenten in (a), (b) und (c) frei von Monosacchariden, insbesondere Glucose, ist, da sonst eine ungewünschte, vorzeitige Enzymreaktion stattfindet. Andererseits weisen die Enzyme in (a), (b) und ggf (c) ihre katalytische Aktivität üblicherweise nur dann auf, wenn ein für ihre Aktivität günstiges Temperaturoptimum erreicht ist. Das Temperaturoptimum für Enzyme liegt üblicherweise in einem Temperaturbereich von mindestens 0 Grad Celsius, das heisst, in einem Temperaturbereich in dem ein zu überwachendes Erzeugnis erfahrungsgemäß aufgetaut vorliegt. Vorzugsweise liegt dieser Temperaturbereich zwischen 0 Grad Celsius und 30 Grad Celsius, besonders bevorzugt im Bereich zwischen 5 Grad Celsius und 25 Grad Celsius und noch bevorzugter zwischen 10 Grad Celsius und 20 Grad Celsius.

Demzufolge eignet sich der erfindungsgemäße Indikator insbesondere für Erzeugnisse, deren Haltbarkeit bekanntermaßen von einer bestimmten Temperatur in einem Bereich von unter 0 Grad Celsius abhängig ist. Jegliche Art von Temperaturschwankungen während der Lagerung kann einen (schnelleren oder langsameren) Farbumschlag hervorrufen.

Darüber hinaus ist dem Fachmann bekannt, dass die Enzyme in (a), (b) und ggf (c) ihre katalytische Aktivität nur in wässrigem Medium aufweisen, das heißt, dass sie in getrocknetem oder in gefrorenem Zustand keine Aktivität aufweisen. Vorzugsweise werden die Komponenten des Indikators nach ihrer Mischung in wasserfreiem, getrocknetem Zustand bewahrt.

Um den Indikator im inaktiven Zustand zu halten, bevor er an den Tiefkühlerzeugnissen angebracht wird, muss der Indikator innerhalb von wenigen Minuten tiefgefroren oder getrocknet werden, so dass der Indikator in einem wasserfreien und/oder in einem für die Aktivität ungünstigen Temperaturbereich vorliegt. Vorzugsweise erfolgt das Einfrieren oder Trocknen innerhalb von 1 bis 10 Minuten, besonders bevorzugt innerhalb von 2 bis 5 Minuten.

Trocknungsverfahren für Enzyme bzw. Proteine sind dem Fachmann aus dem Stand der Technik bekannt. Um Wasser aus einer Proteinlösung zu entfernen muss Wasserdampf erzeugt und aus der Probe abtransportiert werden. Hierfür kennt der Fachmann drei Möglichkeiten: (i) Verdampfung bei Temperaturen oberhalb des Siedepunktes, (ii) Verdunstung unterhalb des Siedepunktes, aber oberhalb des Tripelpunktes von Wasser und (iii) Sublimation aus Eis bei niedrigen Drücken. Prinzip (iii) findet nur bei der Gefriertrocknung Anwendung. Der entstandene Wasserdampf kann abgeführt werden, indem das über dem Gut befindliche Gas in Bewegung versetzt und aus der Trocknungszone entfernt werden. Dies geschieht mittels Verwendung von Ventilatoren, Einblasen trockener Gase, bei gleichzeitiger Absaugung der wasserdampfgesättigten Luft oder durch Evakuierung der Kammer. Mittels eines Eiskondensators kann dafür gesorgt werden, dass sich der Wasserdampf gezielt an einer Stelle abseits des Gutes niederschlägt. Als gängige Trocknungsverfahren kommen für die vorliegende Erfindung in Betracht: Infrarot- oder Mikrowellentrocknung, Konvektionstrocknung, Sprühtrocknung, Gefriertrocknung (Lyophilisation), Vakuumtrocknung, Trocknung mit superkritischen Gase, Sprühgefriertrockung.

Der Trocknungsvorgang sollte äußerst gründlich erfolgen, um auch Spuren verbleibenden Wassers möglichst umfassend zu entfernen. Je nach eingesetztem Trocknungsverfahren kann die gewählte Trocknungstemperatur stark variieren. Beispielsweise bei der Sprühtrocknung können für kurze Zeit -meist wenige Sekunden- Temperaturen bis etwa 150-170°C erreicht werden. Dem Fachmann ist bekannt, welche Temperaturen für das jeweilige Verfahren zweckmäßig sind, ohne dass die zu trocknenden Enzym- bzw. Proteinlösung denaturiert wird.
Für die vorliegende Erfindung ist es besonders günstig, wenn das Polysaccharid als Trägermatrix vorliegt, auf welcher der erfindungsgemäße Indikator aus den Komponenten a), b) und c) zur Temperaturüberwachung aufgebracht ist.

Der Ausdruck "Trägermatrix" umfasst ein beliebiges Material, auf den die Komponenten des Indikators aufgebracht werden können und welches entweder vollständig aus Polysacchariden besteht, oder welches Polysaccharide enthalten kann. Derartige Materialien können synthetische oder natürliche chemische Substanzen oder Substanzen biologischen Ursprungs sein. Die Matrixmaterialien umfassen, ohne darauf beschränkt zu sein, Glas und andere Siliziumoxide, Polystyrol, Polypropylen, Polyethylen, Polyvinylidenfluorid, Polyurethan, Polyalginat, Polysulfon, Polyvinylalkohol, Acrylnitrilpolymere, Polyacrylamid, Polycarbonat, Polypenten, Polypentan, Nylon, Amylosen, Gelatine, modifizierte (z.B. vernetzte) Gelatine, Kollagen, natürliche und modifizierte Polysaccharide wie oben definiert, einschließlich Dextrane und Cellulosen (z.B. Nitrocellulose), Agar und Magnetit. Für die vorliegende Erfindung ist es am günstigsten, wenn das Polysaccharid Cellulose oder Derivate davon ist. Cellulose ist ein natürlich vorkommendes Polysaccharid aus beta-verbundenen Glucose-Einheiten.

Um den Indikator auf der Trägermatrix im inaktiven Zustand zu halten, bevor er an den Tiefkühlerzeugnissen angebracht wird, muss die Trägermatrix, auf welcher der Indikator aufgebracht ist, innerhalb von wenigen Minuten tiefgefroren oder getrocknet werden. Vorzugsweise erfolgt das Einfrieren oder Trocknen innerhalb von 1 bis 10 Minuten, besonders bevorzugt innerhalb von 2 bis 5 Minuten. Um den Trockenvorgang zu beschleunigen, können gängige Verfahren wie Erwärmung, Infrarotbestrahlung, Mikrowellenbehandlung eingesetzt werden.

In einer weiteren Ausführungsform wird der erfindungsgemäße Indikator mittels eines Tintenstrahldruckers, der für Verpackungen geeignet ist, auf die Trägermatrix aufgebracht. Drucksysteme, die für derartige Zwecke in Frage kommen und kommerziell erhältlich sind, werden beispielsweise auf der Internetseite http://www.sigtech-ag.ch/index.php?id=tintenstrahldrucker ausführlich beschrieben.

In einer bevorzugten Ausführungsform des erfindungsgemäßen Indikators handelt es sich bei dem Polysaccharid bzw. bei der Polaysaccharid-Trägermatrix um Cellulose oder Cellulose-enthaltende oder Cellulose-basierte Materialien. Als nicht abschließende Liste von Beispielen hierfür seien wasserlösliche Cellulose-Ether, wie z.B. Carboxymethylcellulose, sowie Alkyl- and Hydroxyalkylcellulosen, beispielsweise Methylcellulose, Hydroxypropoylymethylcellulose, Hydroxyethylcellulose, Hydroxyethylmethylcellulose, Hydroxybutylmethycellulose, und Ethylhydroxyethylcellulose genannt.

Im Allgemeinen umfasst der Begriff Cellulose sämtliche einfach und kommerziell erhältliche Formen von Cellulose, wie z.B. Holzzellstoff, Baumwolle, Hanf, Ramiefaser, oder regenerierte Formen wie z.B. Rayon.

In einer ganz besonders bevorzugten Ausführungsform handelt es sich bei der Polysaccharid-Trägermatrix um Papier.

Die Bezeichnung "Papier" umfasst im Rahmen der vorliegenden Erfindung flächenförmige oder anders geformte Materialien aus Faserstoff oder faserartigem Cellulose-haltigem Material üblicherweise aus natürlicher Herkunft stammend. Papier kann auch aus synthetischen Cellulose-Fasern, regenerierter Cellulose und aus recyclefähigem Abfallpapier hergestellt werden. Darüber hinaus eignen sich für die vorliegende Erfindung Kombinationen von cellulose-haltigen und synthetischen Materialien. Karton fällt ebenfalls unter den Begriff Papier.

Es versteht sich, dass in Abhängigkeit von dem gewählten Polysaccharid unterschiedliche Enzyme oder Enzympräparationen (a), welche die Umsetzung eines Polysaccharids zu einem Monosaccharid katalysieren können, in Frage kommen. Für den erfindungsgemäßen Indikator kommen beispielsweise Cellulasen, Amylasen, Hemicellulasen oder Xylanasen in Betracht. Vorzugsweise werden für die vorliegende Erfindung Cellulasen verwendet. Eine Liste von kommerziell erhältlichen Enzymen, die Polysaccharide abbauen können, ist dem Beispiel 4, Tabelle 4, zu entnehmen.

Es versteht sich außerdem, dass in Abhängigkeit von dem unter (a) entstehenden Monosaccharid unterschiedliche Enzyme oder Enzympräparationen (b), welche die Oxidation eines Monosaccharids katalysieren können, in Frage kommen. Für den erfindungsgemäßen Indikator kommen beispielsweise Malat Oxidase (EC 1.1.3.3), Glukose Oxidase (EC 1.1.3.4), Hexose Oxidase (EC 1.1.3.5), L-gulonolactone oxidase (EC 1.1.3.8), galactose oxidase (EC 1.1.3.9), pyranose oxidase (EC 1.1.3.10), L-sorbose oxidase (EC 1.1.3.11), N-acylhexosamine oxidase (EC 1.1.3.29), D-Arabinono-1,4-lactone oxidase (EC 1.1.3.37), D-mannitol oxidase (EC 1.1.3.40) , xylitol oxidase (EC 1.1.3.41), cellobiose dehydrogenase (EC 1.1.99.18), aldehyde oxidase (EC 1.2.3.1) in Betracht.

In einer bevorzugten Ausführungsform ist das Enzym (b) welches die Oxidation eines Monosaccharids katalysiert, eine Glucose-Oxidase. Die Bezeichnung "Glucose-Oxidase" bezieht sich auf ein Oxidase-Enzym der Klasse EC 1.1.3.4, ein dimeres Protein, welches die Oxidation von beta-D-Glucose in D-Glucono-1,5-Lacton katalysiert, wobei letzteres zu Gluconsäure hydrolysiert und gleichzeitig molekularer Sauerstoff zu Wasserstoffperoxid (H₂O₂) reduziert wird. Wasserstoffperoxid wiederum oxidiert unter Einwirkung des zweiten Enzyms, wie im folgenden erläutert, einen Stoff der dabei die Farbe ändert (Redoxindikator).

Für Funktion des Indikators ist es erforderlich, dass der Indikator außer den Enzymen a) und b) noch ein Gemisch c) umfasst, welches zum Nachweis der Enzymaktivität aus a) und/oder b) geeignet ist.

Dieses Gemisch enthält erfindungsgemäß einen Farbindikator, wie definiert, und üblicherweise ein entsprechendes Indikator-Enzym, welches in der Lage ist, einen Farbumschlag des Farbindikators zu bewirken.

Unter einem "Indikator-Enzym" wird jedes Enzym verstanden, dessen Enzymaktivität durch Reaktion mit einem oder mehreren Substraten für dieses Enzym bestimmt werden kann. Insbesondere können alle bekannten Indikatorreaktionen, welche eine Enzym-Substrat-Reaktion ausnützen, verwendet werden. Dazu gehören beispielsweise solche, bei denen direkt ein detektierbares Produkt entsteht, aber auch solche, bei denen das Produkt der Reaktion erst in weiteren Reaktionsschritten zu einem detektierbaren Signal führt. Substrat in solchen Reaktionen, an die sich weitere Reaktionsschritte anschließen, kann beispielsweise wiederum ein Trägermaterial sein, an welches ein weiteres Indikatorenzym gebunden ist. Besonders zweckmäßig sind solche Reaktionen, in deren Verlauf eine Farbänderung stattfindet oder eine farbige Verbindung gebildet wird oder verschwindet. Solche Enzyme und dazugehörige Substrate sind dem Fachmann bekannt. Beispiele für Indikatorenzyme/Substrate sind β-Galactosidase/β-Galactoside, Peroxidase/Peroxide, sowie Phosphatasen/Phosphate usw.

Außerdem enthält es zweckmäßigerweise die Reagenzien, die zur Bestimmung der Konzentration des Indikatorenzyms erforderlich sind. Diese Reagenzien enthalten beispielsweise ein Indikatorenzymsubstrat oder chromogenes Substrat wie oben definiert.

Indikatorenzymsubstrate bzw. chromogene Substrate sind Verbindungen, welche durch Katalyse durch das Indikatorenzym eine nachweisbare Änderung vollziehen. Sie sind bevorzugt spaltbar oder Bestandteile eines Redoxsystems. Der Nachweis des Substrats beziehungsweise des Produkts seiner Reaktion mit dem Indikatorenzym kann beispielsweise colorimetrisch, fluorimetrisch oder auch elektrochemisch vorgenommen werden.

Außerdem können die Reagenzien erforderlichenfalls pH-Puffersubstanzen, Stabilisatoren, Aktivatoren etc. enthalten. Die Art des Indikatorenzymsubstrats sowie die weiteren Inhaltsstoffe richten sich nach dem zu bestimmenden Indikatorenzym und sind dem Fachmann bekannt.

In einer ganz besonders bevorzugten Ausführungsform ist das Indikator-Enzym eine Peroxidase und folglich das chromogene Substrat ein Redoxindikator für eine Peroxidase (siehe Definitionen und Tabelle 2).

Bevorzugt wird als Farbstoff ABTS eingesetzt. Die Konzentration des ABTS wird dabei so gewählt, dass sie der oberen Grenze des Messbereiches entspricht. Dadurch ändert sich mit zunehmender Enzymkonzentration und entsprechend zunehmendem H₂O₂ -Umsatz nicht nur die Konzentration eines farbigen Produktes, sondern auch das Verhältnis zweier unterschiedlich gefärbter Produkte und dadurch die spektrale Zusammensetzung der zu betrachtenden Farbe. Vorzugsweise liegt die Konzentration von ABTS in einem Bereich von 0,05mg/ml bis 10mg/ml, besonders bevorzugt in einem Bereich zwischen 0,5 und 7mg/ml, ganz besonders bevorzugt in einem Bereich zwischen 0,7 und 3mg/ml.

Im Rahmen der vorliegenden Erfindung wird eine Oxidase in einem gekoppelten System mit der Peroxidase verwendet. Wie oben beschrieben setzt die Glucose-Oxidase Glucose mit Sauerstoff zu Gluconsäure um, wobei H₂O₂ als Nebenprodukt entsteht, dessen Konzentration mit einem chromogenen Substrat wie ABTS und Peroxidase bestimmt wird. Dabei kann die Peroxidase in so hohen Konzentrationen eingesetzt werden, dass das H₂O₂ innerhalb weniger Minuten - und bis zu mehreren Stunden- quantitativ umgesetzt wird, sobald der Indikator aktiviert ist.

In einer anderen Ausführungsform ist das Indikator-Enzym des Gemisches (c) eine Perhydrolase und folglich das chromogene Substrat ein Redoxindikator für eine Perhydrolase (siehe Definitionen und Tabelle 1).

Als "Hydrolasen", insbesondere "Perhydrolasen", die auch unter "Haloperoxidasen" bekannt sind, bezeichnet man Enzyme, die in Anwesenheit von Wasserstoffperoxid (H₂O₂) die Bildung von ausreichenden Mengen an Persäuren katalysieren um einhergehende Halogenierungsreaktionen zu ermöglichen (Enzyme der Klassifikation EC 3.). Wird für den Indikator eine Perhydrolase verwendet, enthalten die Reagenzien in (c) weiterhin Halogenide, vorzugsweise Kaliumbromid, und einen Farbindikatior, vorzugsweise Phenolrot. Alternativ können Persäuren mit ABTS und Kaliumiodid nachgewiesen werden (Pinkernell, U. et al., Analyst, 1997, 122, 567-71), sowie Acetat als Puffersystem oder einen Carbonsäureester-Derivat. Im Fall von Carbonsäureester-Derivaten, können Lipasen, Proteasen oder Esterasen verwendet werden, die als Nebenreaktion bekanntlich eine Perhydrolyse katalysieren können (WO 2009/067279; WO 2006133079; Molecular basis of perhydrolase activity in serine hydrolases, Angewandte Chemie, International Edition (2005), 44(18), 2742-2746; Molecular cloning and expression of perhydrolase genes from Pseudomonas aeruginosa and Burkholderia cepacia in Escherichia coli, Biotechnology Letters (2006), 28(12), 849-856).In einer bevorzugten Ausführungsform wird eine Perhydrolase aus Flavobacterium johnsoniae UW101 (EC 3.1.1.), siehe SEQ ID NO:1, verwendet.

Um ausreichende Mengen des Enzyms zu erhalten, kann eine für die SEQ ID NO.1 kodierende Nukleinsäure in Bakterien, vorzugsweise in Escherichia coli, exprimiert und aufgereinigt werden. Verfahren zum Exprimieren und Aufreinigen von heterolog exprimierten Proteinen in Bakterien sind dem Fachmann hinlänglich bekannt (siehe insbesondere "Molecular Cloning: A Laboratory Manual"; Sambrook and Russell, CSHL Press 2001, ISBN 978-087969577-4).

Ein weiterer Gegenstand der vorliegenden Erfindung betrifft ein Verfahren zum Herstellen eines Indikators zur Temperaturüberwachung eines gekühlten oder tiefgekühlten Erzeugnisses, umfassend das Mischen
a. mindestens eines Enzyms, das die Umsetzung eines Polysaccharids zu einem Monosaccharid katalysiert, mit
b. mindestens einem Enzym, das die Oxidation des mittels a) gebildeten Monosaccharids katalysiert,
   und mit
c. einem Gemisch, das zum Nachweis der Enzymaktivität aus a) und/oder b) geeignet ist und das einen Farbindikator enthält.

Vorzugsweise umfasst das Verfahren zusätzlich das Aufbringen der Komponenten a), b) und c) auf eine Polysaccarid-Trägermatrix.

In einer weiteren Ausführungsform wird der Indikator direkt auf die Verpackung des zu gefrierenden Erzeugnisses aufgebracht. Verfahren zum Aufbringen von Labels aus verschiedenen Materialien, wie zum Beispiel Papier, auf Verpackungen sind dem Fachmann geläufig. Üblicherweise werden Etiketten mittels Etikettiermaschinen aufgebracht, die die Etiketten entweder mechanisch oder manuell oder mittels Luftdruck auf die Verpackung aufbringen. Siehe unter:
http://www.bluhmsysteme.com/
sowie unter
www.neue-verpackung.de/ai/resources/0705dceb58a.pdf

Ist die Verpackung aus Papier oder Karton, wie bei gängigen Tiefkühlprodukten wie Pizza, TK-Gemüse, TK-Obst der Fall, dient die Verpackung selbst als Polysaccharid-Trägermatrix.

Zur Lagerung bzw. zur Vermeidung einer vorzeitigen Aktivierung des Indikators wird die Polysaccharid-Trägermatrix nach Aufbringen der Komponenten a), b), c) sofort getrocknet oder sofort tiefgefroren. Gängige Trockungsverfahren wurden oben beschrieben.

Ein weiterer Gegenstand der vorliegenden Erfindung betrifft ein Verfahren zur Temperaturüberwachung eines gekühlten oder tiefgekühlten Erzeugnisses, umfassend
i. das Aufbringen eines erfindungsgemäßen Indikators auf das Erzeugnis,
ii. und das Kontrollieren der Enzymaktivität anhand des Farbindikators,
wobei der positive Nachweis der Enzymaktivität indikativ für eine Unterbrechung der Kühlung oder Tiefkühlung des Erzeugnisses ist.

Im Falle einer Unterbrechung der Kühlkette, d.h. wenn das Erzeugnis in unerwünschter Weise aufgetaut wird, wird die den Indikator enthaltende Polysaccharid-Trägermatrix durch die beim Auftauen entstehende Flüssigkeit feucht bzw. wässrig und in einen Temperaturbereich überführt, bei welchen die Enzyme (a), (b) und (c) des erfindungsgemäßen Indikators ihre katalytische Aktivität aufweisen, so dass die oben beschriebene Enzymkaskade, die sich anhand des Farbumschlags des Farbindikators nachweisen lässt, gestartet wird.

In einer bevorzugten Ausführungsform erfolgt das Aufbringen unter Bedingungen, bei welchen keine Enzymaktivität nachweisbar ist. Demnach kann das Erzeugnis im nicht gekühlten Temperaturbereich verpackt werden, der Indikator auf die Verpackung aufgebracht und beides zusammen tiefgekühlt werden. Alternativ kann ein bereits tiefgekühltes Erzeugnis in eine Verpackung, welche den erfindungsgemäßen Indikator umfasst, gefüllt werden. Anschließend wird das Erzeugnis wieder unter Tiefkühlbedingungen gelagert.

Ein weiterer Gegenstand der Erfindung betrifft eine Verpackung zur Aufbewahrung eines gekühlten oder tiefgekühlten Erzeugnisses, dadurch gekennzeichnet, dass die Verpackung mit wenigstens einem erfindungsgemäßen Indikator, der ortsfest oder entfernbar sein kann, versehen ist.

Der Ausdruck "Verpackung" bezeichnet ein Behältnis, welches geeignet ist, Verbrauchsprodukte oder Erzeugnisse aufzubewahren, wobei das Behältnis nicht auf eine bestimmte Form oder Größe beschränkt ist und aus Metall, Glas, Plastik, Polystyren, Papier (Karton) oder Mischformen daraus hergestellt sein kann. Als Verpackung kommen Dosen, Kartons, Schale, Ablagen, Taschen, Umschläge, Einwickelfolien, Frischhaltefolien und ähnliches in Betracht.

Vorzugsweise ist der Indikator nach außen sichtbar auf der Verpackung angeordnet.

Sofern das Verpackungsmaterial nicht bereits selbst aus Polysacchariden besteht oder selbige enthält wie Papiermaterialien, wird die Verpackung mit einem Indikator, der zuvor auf eine Polysaccharid-Trägermatrix aufgebracht worden ist, versehen.

Der erfindungsgemäße Indikator kann mittels einer Reihe von Methoden, die dem Fachmann bekannt sind, auf die Verpackung, beispielsweise wie ein Etikett, aufgebracht werden. Zum Schutz des Indikators kann der Indikator dabei wenigstens zwischen einer äußeren, sichtbaren und/oder UV- Licht durchlässigen, vorzugsweise für Wasser undurchlässigen, Schicht und/oder einer für den Indikator undurchlässigen, dem Erzeugnis zugewandten, inneren Schicht angeordnet sein. Wenn der Indikator entfernbar sein soll, kann er in einem kleinen Beutel mit einer äußeren und einer inneren Schicht aus gängigen Verpackungsmaterialien bereitgestellt werden. In ein geeignetes Material in Form eines kleinen Beutels oder Umschlags wird eine Polysacccarid-Trägermatrix enthaltend den Indikator platziert. Der Beutel wird sodann verschlossen durch Zuschweißen oder andere geeignete Verfahren und kann dann auf die Packung aufgebracht werden. Zum Aufbringen eignen sich adhäsive Klebematerialien, in Form von Klebestreifen, die üblicherweise für derartige Zwecke verwendet werden.

Die äußere, der Betrachtung zugewandte Schicht sollte aus einem flexiblen, durchsichtigen Plastikfolienmaterial bestehen. Derartige Materialien sind dem Fachmann bekannt und umfassen Materialien wie Polyester, Polycarbonat, Polyethylen, Polypropylen, Polyamid, Polyurethan, Polyvinylchlorid, oder ähnliches.

Der Indikator kann auf eine frische Erzeugnisverpackung aufgebracht oder darin eingeschlossen werden, bevor die Verpackung enthaltend das Erzeugnis eingefroren wird. Das Erzeugnis wird anschließend eingefroren, und der Indikator zeigt an, ob die Verpackung bzw. das Erzeugnis aufgetaut vorlag, selbst wenn es nach dem Auftrauen wieder eingefroren wurde.

Ein weiterer Gegenstand der Erfindung betrifft die Verwendung einer erfindungsgemäßen Verpackung zum Aufbewahren von gekühlten oder tiefgekühlten Erzeugnissen. Tiefgekühlte oder gekühlte Erzeugnisse können Blutkonserven, Lebensmittel, Impfstoffe, Arzneimittel, Blutprodukte, Implantate, Diagnostika, Augentropfen, Kontaktlinsen und Chemikalien umfassen. Je nach Erzeugnis ist die Art der Verpackung zu wählen.

Besondere Vorteile der vorliegenden Erfindung liegen darin, dass die ideale Zusammensetzung durch den Einsatz von angepassten Enzym- und Substrat-Konzentrationen erreicht werden kann. Das System ist sehr flexibel einsetzbar und seine Komponenten - inkl. der Enzyme - sind kostengünstig zu erwerben. Es ist möglich, einen inerten Indikator als Flüssig-Formulierung anzubieten, die beim Verpackungsprozess nur noch auf die Verpackung gedruckt werden muss. Außerdem kann die Enzym Formulierung auf ein glukosefreies Label aufgetragen und dieses getrocknet werden, so dass ein lagerfähiges Label bereitgestellt wird. Zur Aktivierung würde der Anwender bzw. Fachmann (z.B. Verpackungshersteller, Lebensmittelhersteller) Wasser auftragen und das Label anschließend mit einer Schutzfolie aus für den Fachmann geläufigen Verpackungsmaterialien abdecken.

Die Erfindung wird anhand der nachfolgenden Beispiele näher erläutert, ohne darauf beschränkt zu sein.

### BEISPIELE

### Beispiel 1: Erfindungsgemäße Zusammensetzungen

### Smart Ink für das Peroxidase Label:

- Cellulase Onozuka RS aus Trichoderma viride (Serva) - 2 U/mg - Glukosefrei (β-Glukosidase aus Mandeln (Sigma) - 2,52 U/mg - Glukosefrei)
- Glukose Oxidase aus Aspergillus niger Typ 11 (Sigma) - 15,5 U/mg
- Peroxidase aus der Sojabohne (Sigma) - 90 U/mg (purpurgallin)
- ABTS (Roche)

### Smart Ink für das Perhydrolase Label:

- Cellulase Optimash BG (Genencor) - 35 mg/mL
- Glukose Oxidase aus Aspergillus niger Typ 11 (Sigma) - 15,5 U/mg
- Perhydrolase aus Flavobacterium johnsoniae - 1,5 mg/mL
- Kaliumbromid & Phenolrot

Alle Komponenten sind als stabile Stocklösung (Smart Ink) in Na-Acetat Puffer (pH 5.5) formuliert.

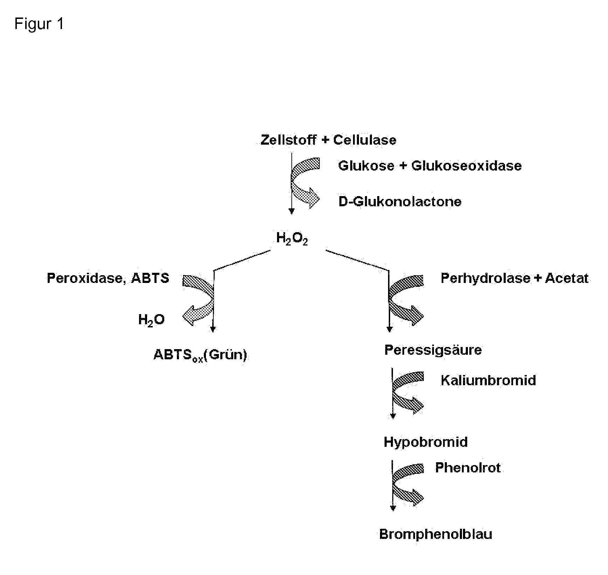

Durch Zugabe der beschriebenen Smart Ink Formulierungen auf einer Polysaccharid-Trägermatrix, insbesondere Zellstoff/Cellulse (gebl. Birkensulfat Papier - Glukosefrei) wird die enzymatische Kaskade aktiviert (s. Figur 1)

### Beispiel 2: Peroxidase-Label

Das Peroxidase Label funktioniert mittels Freisetzung von Glukose aus Zellstoff durch Cellulasen. Glukoseoxidase oxidiert die freigewordene Glukose zu D-Glukonolactone und H₂O₂. Eine Peroxidase oxidiert mit Hilfe von H₂O₂ das Modell-Substrat ABTS in seine chromophore Form. Man erhält eine Farbveränderung von farblos/hellgrün nach dunkelgrün.

Von der Onozuka-Cellulase und der ß- Glukosidase wurden Stammlösungen mit jeweils 1000 U/mL hergestellt. Im Fall einer Kombination von Onozuka und ß- Glukosidase wurde ein Verhältnis von 4:1 gewählt. In dieser Mischung sind 800 U/mL Onozuka RS und 200 U/mL β-Glukosidase enthalten.

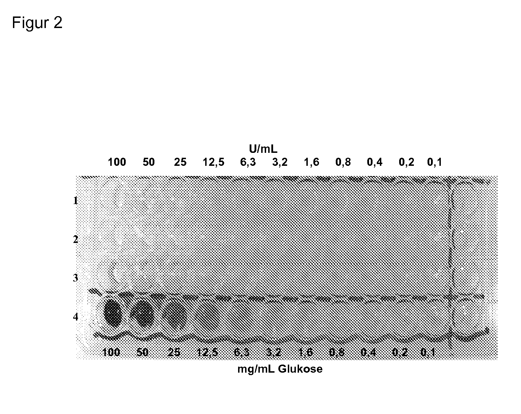

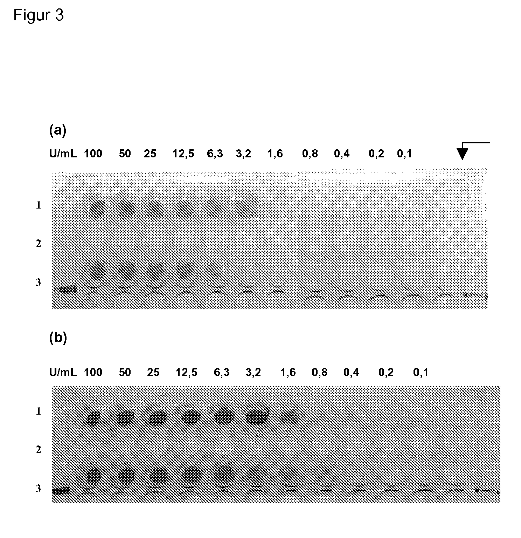

Die verschiedenen Enzym-Lösungen wurden in einer Mikrotiterplatte jeweils von links nach rechts 1:2 verdünnt. Von den einzelnen Verdünnungen wurden je 20 µL zu 180 µL Reaktionslösung gegeben (= Smart Ink MTP, Figur. 2). Von der Smart Ink MTP wurden - pro Verdünnung - 30 µL auf eine MTP mit Birkensulfatplättchen zugegeben (= Zellstoff MTP, s. Figur. 3).

### Smart lnk für das Peroxidase Label:

| | |
|---|---|
| 100 mM Na-Acetat (pH 5,5) | 180µL |
| 10 µg/mL Peroxidase | |
| 10 µg/mL Glukose Oxidase | |
| 1 mg/mL ABTS | |
| Cellulase(n) - 1:2 Verdünnungsreihe von links nach rechts | 20µL |

Die erste und die dritte Zeile der Mikrotiterplatte aus Figur 2 zeigen die fertige Smart Ink Formulierung. Sogar bei den höchsten Enzymkonzentrationen ist, auch nach zwei Tagen, keine Grünfärbung der Lösung sichtbar. Die leicht gelbliche Farbe der Lösung ist auf die Eigenfarbe der Cellulase-Stocklösung und nicht auf Anwesenheit von Glukose zurückzuführen (s. zweite Zeile mit nur β- Glukosidase). In der unteren Zeile ist eine Glukoseverdünnungsreihe dargestellt, um die Sensitivität des Systems zu quantifizieren. In der rechten Spalte befindet sich die Negativ-Kontrolle (Wasser statt Zellulase). Hier findet keine Reaktion statt, weder in der Smart Ink MTP noch in der Zellstoff MTP (s. Figur 2).

Die Zellstoff MTP (Figur 3) zeigt, dass die Cellulase Onozuka RS den Zellstoff sehr effektiv hydrolysieren konnte und in der Lage war, genügend Glukose für die weitere enzymatische Reaktionsschritte der Kaskade freizusetzen. Eine erste Verfärbung setzte bereits nach 15 Minuten ein (RT, hier nicht gezeigt). Nach 30 Minuten war der Grad der Verfärbung der Zellstoffstückchen schon weit fortgeschritten (bis 1,6 U/mL Onozuka RS). Die ß- Glukosidase allein konnte aus dem verwendeten Zellstoff keine Glukose freisetzen und somit keine Färbung des ABTS über die Enzymkaskade auslösen. Die Mischung der Onozuka und der ß- Glukosidase zeigte keine Verbesserung des Systems und ist daher nicht unbedingt notwendig.

Bei einem Parallelversuch wurde eine frisch behandelte Zellstoff MTP innerhalb von 2-5 Minuten bei -20°C eingefroren. Nach 5 Tage wurde die Zellstoffplatte bei aufgetaut. Die Grünfärbung wurde erst nach ca. 40 Minuten in Raumtemperatur deutlich sichtbar. Damit konnte das Anwendungskonzept für einen Gefrierindikator bewiesen werden.

Beispiel 3: Testung verschiedener Enzyme, die den Abbau / die Umwandlung von Polysacchariden zu Monosacchariden katalysieren können.

Ein Screening von kommerziell erhältlichen Cellulasen wurde durchgeführt. Dafür wurden 40, für das System interessante, Kandidaten untersucht (u.a. Cellulasen, Cellubiasen, Amylasen, Hemicellulasen und Xylanasen - s. Anhang). Alle Enzyme wurden in einem Vorversuch auf Anwesenheit von Glukose getestet. Dabei wurden die entsprechenden Smart Ink Lösungen vorbereitet und über Nacht bei RT inkubiert. Das bedeutet, dass 20 µL aus der Stammlösung jedes Kandidaten in 180 µL der Reaktionslösung gegeben wurden.

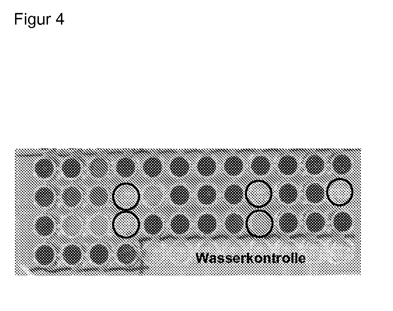

Aus dieser eher qualitativer Analyse wurden 5 Enzym-Formulierungen identifiziert, die absolut glukosefrei waren (s. Figur 4, eingekreiste Mikrotiterplatten-Vertiefungen - o). Bei den restlichen 35 Formulierungen zeigt sich eine leichte bis starke Grünfärbung, was auf die - für diese Anwendung - eher ungünstige Anwesenheit von Glukose hindeutet.

Von den fünf glukosefreien Enzym-Formulierungen hatten zwei auf Zellstoff MTP eine positives Signal ergeben: die Novozyme Cellulasen Novo 342 & Novo 613.

Die restlichen 35 glukosehaltige Formulierungen wurden mit einer desalting Säule (Hi-Trap, Fa. GE - isokratischen Lauf, 100 mM Na-Acetat pH 5,5) gereinigt und danach auf einer Zellstoff MTP getestet. So konnten zusätzlich 18 Enzym-Formulierungen als positiv identifiziert werden.

**Tabelle 3.**

| Nr. | Name | Firma | Enzymklasse |
|---|---|---|---|
| 1 | BAN 480L | Novozym | Glucanohydrolase |
| 2 | Celluclast 1,5 FG | Novozym | Cellulase |
| 3 | CelluFood AL 140 | Biopract | β-Glucanase, Cellulase, Xylanase |
| 4 | Cellulase | Sigma | Cellulase |
| 5 | Denimax 16000L | | |
| 6 | Dextrozym GA | Novozym | Gluco- Amylase |
| 7 | DP10 | Delta Ingridients | Cellulase |
| 8 | Fermenzyme L400 | Genencore | Gluco- Amylase Protease |
| 9 | Glucanase 1XL-G011L | Biocatalysts (Wales) | β-Glucanase, Xylanase, Cellulase |
| 10 | Glucanase 5XL-G015L | Biocatalysts (Wales) | β-Glucanase, Xylanase, Cellulase |
| 11 | Glucanex 200G | Novozymes | Glucanase, Protease, Chitinase, Cellulase |
| 12 | G-Zyme 480 Ethanol | Genencore | Gluco- Amylase |
| 13 | Liquozym SC DS | Novozym | a- Amylase |
| 14 | Multifect CX 2000L | Genencor (NL) | Cellulase |
| 15 | Novo 188 | Novozym | Cellubiase |
| 16 | Novo 342 | Novozym | Cellulase Hemicellulase |
| 17 | Novo 476 | Novozym | Cellulase |
| 18 | Novo 50045 | Novozym | - |
| 19 | Novo 51008 | Novozym | Cellulase |
| 20 | Novo 51081 | Novozym | Cellulase |
| 21 | Novo 613 | Novozym | Endo-Glukanase |
| 22 | Optimash BG | Genencore | β-Glucanase Xylanase |
| 23 | Optimash XL | Genencore | Cellulase Xylanase |
| 24 | Pulpzym HC | Novozym | Xylanase |
| 25 | Shearzyme 500L | Novozym | Xylanase |
| 26 | Spezyme Ethyl | Genencore | a- Amylase |
| 27 | Spezyme Fred | Genencore | a- Amylase |
| 28 | Spezyme HPA | Genencore | a- Amylase |
| 29 | Spirizym Fuel | Novozym | Gluco-Amylase |
| 30 | ß-Glucanase Sub-mers | Erbslöh | Glucanase wenig Laminarainase Cellulase |
| 31 | Stargen 001 | Genencore | Amylase |
| 32 | Themamyl SC | Novozym | a- Amylase |
| 33 | Ultra Thin | Valley Research | a- Amylase |
| 34 | Viscoflow L | Novozymes | Glucanase, Xylanase, Cellulase |
| 35 | Viscozyme BG | Novozym | Cellulase Xylanase |
| 36 | Viscozyme L | Novozym | |
| 37 | Viscozyme Wheat | Novozym | Enzymmix |
| 38 | VP 0965/4 ex T.reesii | Erbslöh | Xylanase, Pektinase,Cellulase, Galoaktomannase |
| 39 | WXL 600 | Delta Ingridients | - |
| 40 | Xylanase | BASF | Xylanase |

Insgesamt konnten in den oben beschriebenen Versuchen 21 funktionsfähige und kommerziell erhältliche Enzyme identifiziert werden. Diese Vorversuche zeigen eindeutig, dass die entsprechenden Smart Ink Formulierungen für das Peroxidase Label auch mit unterschiedlichen Cellulasen funktionsfähig sind. Dies sollte für spätere Enzymkombinationen einen Ansatzpunkt für sehr flexible und kostengünstige Formulierungen bieten.

### Beispiel 4: Perhydrolase Label

Bei dem Perhydrolase Label erfolgt ebenfalls eine Freisetzung von Glukose aus Zellstoff durch Cellulasen. Die Glukoseoxidase oxidiert wieder die freigewordene Glukose zu D-Glukonolactone und H₂O₂. Im nächsten Schritt wird Acetat - als konzentriertes Puffersystem benutzt - durch eine Perhydrolase mit H₂O₂ perhydrolysiert. Die so entstandene Peressigsäure oxidiert in situ Kaliumbromid zu Kaliumhypobromid, was wiederum Phenolrot zu Bromphenolblau bromiert. Man erhält eine Farbveränderung von gelb nach blau.

Von der Perhydrolase wird eine Stammlösung von 150 µg/mL hergestellt. Für die Perhydrolaseversuche wurden zwei Cellulasen getestet. Zum einen die Zellulase Onozuka RS, die sich bei den Versuchen zum Peroxidase Label als sehr effektiv erwiesen hat und zum andern die Glukanase/Xylanase Mischung Optimash BG (Fa. Genencor), die sich nach dem Entsalzen im Zellstoff MTP Test ebenfalls aktiv war. Testversuche zeigten allerdings, dass nur die Glukanase/Xylanase Mischung Optimash BG in diesem System funktionsfähig war.

### Smart Ink für das Perhydrolase Label:

| | |
|---|---|
| 1 M | Na-Acetat (pH 5,5) |
| 100 µg/mL | Glukose Oxidase |
| 450 mM | KBr |
| 44 oder 88 µM | Phenol Rot |

| | |
|---|---|
| + Optimash BG + Perhydrolase aus F. johnsoniae | |

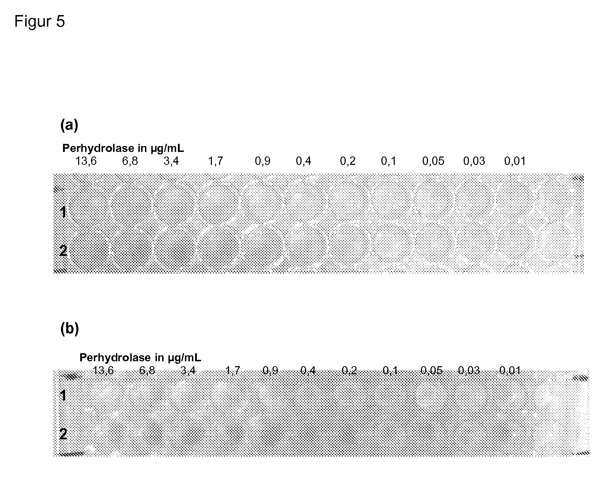

Die Perhydrolase Stammlösung (150 µg/mL) wurde in einer Mikrotiterplatte von links nach rechts 1:2 verdünnt. Von den einzelnen Verdünnungen wurden je 20 µL Perhydrolaseverdünnung und 20 µL Optimash BG (Gesamtproteinkonzentration 13,3 mg/mL) zu 180 µL Reaktionslösung dazugegeben. Von der Smart Ink MTP wurden - pro Verdünnung - 30 µL auf eine MTP mit Birkensulfatplättchen zugegeben (= Zellstoff MTP; s. Figur 5).

Bereits nach 1,5 h bei Raumtemperatur kann man eine deutliche Verfärbung des Zellstoffs der beiden höchsten Konzentrationen der Perhydrolase erkennen. Die Konzentration von Phenolrot ist für die Sensitivität des Systems nicht maßgeblich entscheidend (s. Figur 5). Als Kontrolle wurde, wie in den oben beschriebenen Versuchen Wasser, statt Perhydrolase und Optimash BG, zu der Reaktionslösung zugegeben. Diese Wasserkontrolle blieb gelb (Figur 5, ganz rechts). Als zweite Kontrolle wurde in diesem Versuch zu der Reaktionslösung nur 20 µL Optimash BG dazugegeben (ohne Perhydrolase) und von dieser Formulierung 30 µL auf die Zellstoff MTP gegeben. Dabei erhält man nach 48 h eine sehr helle Blaufärbung, was vermutlich auf die Anwesenheit des entstehenden H₂O₂ zurückzuführen ist. Da die Zeit in der das Perhydrolase-Label eine erste Farbveränderung anzeigt unter 2 Stunden liegt, ist diese Nebenreaktion vernachlässigbar.

## Patentansprüche

1. Indikator zur Temperaturüberwachung eines gekühlten oder tiefgekühlten Erzeugnisses, umfassend die Komponenten:
a) mindestens ein Enzym, das die Umsetzung eines Polysaccharids zu einem Monosaccharid katalysiert,
b) mindestens ein Enzym, das die Oxidation des mittels a) gebildeten Monosaccharids katalysiert,
und
c) ein Gemisch, das zum Nachweis der Enzymaktivität aus a) und/oder b) geeignet ist und das einen Farbindikator enthält,
**dadurch gekennzeichnet, dass** die Enzymaktivität erst nach Aufbringen auf eine Polysaccharid-Trägermatrix in Anwesenheit von Wasser und in einem Temperaturbereich, bei dem das Erzeugnis aufgetaut vorliegt, nachweisbar ist.

2. Indikator nach Anspruch 1, **dadurch gekennzeichnet, dass** der Temperaturbereich zwischen 0 Grad Celsius und 30 Grad Celsius, vorzugsweise im Bereich zwischen 5 Grad Celsius und 25 Grad Celsius und noch bevorzugter zwischen 10 Grad Celsius und 20 Grad Celsius liegt.

3. Indikator nach Anspruch 1 oder 2, **dadurch gekennzeichnet, dass** die Komponenten a), b) und c) auf einer Polysaccharid-Trägermatrix aufgebracht sind.

4. Indikator nach einem der Ansprüche 1 bis 3, **dadurch gekennzeichnet, dass** das Monosaccharid Glucose ist.

5. Indikator nach einem der Ansprüche 1 bis 4, **dadurch gekennzeichnet, dass** das Polysaccharid Cellulose ist.

6. Indikator nach einem der Ansprüche 1 bis 5, **dadurch gekennzeichnet, dass** der Farbindikator ein chromogenes Substrat für eine Peroxidase umfasst.

7. Indikator nach einem der Ansprüche 1 bis 6, **dadurch gekennzeichnet, dass** der Farbindikator ein chromogenes Substrat für eine Hydrolase, insbesondere eine Perhydrolase umfasst.

8. Verfahren zum Herstellen eines Indikators zur Temperaturüberwachung eines gekühlten oder tiefgekühlten Erzeugnisses, umfassend das Mischen
a) mindestens eines Enzyms, das die Umsetzung eines Polysaccharids zu einem Monosaccharid katalysiert, mit
b) mindestens einem Enzym, das die Oxidation des mittels a) gebildeten Monosaccharids katalysiert,
und mit
c) einem Gemisch, das zum Nachweis der Enzymaktivität aus a) und/oder b) geeignet ist und das einen Farbindikator enthält.

9. Verfahren nach Anspruch 8, **dadurch gekennzeichnet, dass** das Verfahren zusätzlich das Aufbringen der Komponenten a), b) und c) auf eine Polysaccharid-Trägermatrix umfasst.

10. Verfahren zur Temperaturüberwachung eines gekühlten oder tiefgekühlten Erzeugnisses, umfassend
(i) das Aufbringen eines Indikators, wie in den Ansprüchen 1 bis 7 definiert, auf das Erzeugnis,
(ii) und das Kontrollieren der Enzymaktivität anhand des Farbindikators,
wobei der positive Nachweis der Enzymaktivität indikativ für eine Unterbrechung der Kühlung oder Tiefkühlung des Erzeugnisses ist.

11. Verfahren nach Anspruch 10, **dadurch gekennzeichnet, dass** das Aufbringen unter Bedingungen erfolgt, bei welchen keine Enzymaktivität nachweisbar ist.

12. Verpackung zur Aufbewahrung eines gekühlten oder tiefgekühlten Erzeugnisses, **dadurch gekennzeichnet, dass** die Verpackung mit wenigstens einem ortsfesten oder entfernbaren Indikator, wie in den Ansprüche 1 bis 7 definiert, versehen ist.

13. Verpackung nach Anspruch 12, **dadurch gekennzeichnet, dass** der Indikator nach außen sichtbar angeordnet ist.

14. Verpackung nach Anspruch 12 oder 13, **dadurch gekennzeichnet, dass** der Indikator wenigstens zwischen einer äußeren, sichtbaren und/oder UV- Licht durchlässigen, vorzugsweise Wasser-undurchlässigen, Schicht und/oder einer für den Indikator undurchlässigen, dem Erzeugnis zugewandten, inneren Schicht angeordnet ist.

15. Verwendung einer Verpackung, wie in einem der Ansprüche 12 bis 14 definiert, zur Aufbewahrung von gekühlten oder tiefgekühlten Erzeugnissen ausgewählt aus der Gruppe bestehend aus Lebensmitteln, Blutkonserven, Impfstoffen, Arzneimittel, Blutprodukten, Implantaten, Diagnostika, Augentropfen, Kontaktlinsen und Chemikalien.


SEQUENCE LISTING

<110>  BASF SE

<120>  Zusammensetzung und Verfahren zur Temperaturüberwachung von Kühl-
        und Tiefkühlerzeugnissen

<130>  PF62853

<160>  1

<170>  PatentIn version 3.4

<210>  1
<211>  271
<212>  PRT
<213>  Flavobacterium

<400>  1

Met Ser Thr Phe Ala Val Asn Asp Gly Thr Glu Ile Tyr Tyr Lys Asp
1               5                   10                  15

Trp Gly Thr Gly Gln Pro Ile Val Phe His His Gly Trp Pro Leu Ser
            20                  25                  30

Ser Asp Asp Trp Asp Ala Gln Met Met Phe Phe Leu Gln Lys Gly Tyr
        35                  40                  45

Arg Val Ile Ala His Asp Arg Arg Gly His Gly Arg Ser Gly Gln Ser
    50                  55                  60

Ser Glu Gly Asn Asn Met Glu Thr Tyr Ala Ser Asp Ile Ala Glu Leu
65                  70                  75                  80

Thr Glu Ala Leu Asn Leu Lys Asp Ala Ile His Val Gly His Ser Thr
                85                  90                  95

Gly Gly Gly Glu Val Ile Arg Tyr Ala Ala Lys Tyr Gly Lys Gly Arg
            100                 105                 110

Ile Ala Lys Ala Val Ile Ile Ser Ala Val Thr Pro Ile Met Ile Gln
        115                 120                 125

Asn Glu Ser Asn Pro Glu Gly Val Pro Leu Ser Val Phe Asp Glu Ile
    130                 135                 140

Arg Gln Gly Thr Gly Phe Asn Arg Ala Gln Tyr Phe Tyr Asp Phe Pro
145                 150                 155                 160

Thr Ala Phe Tyr Gly Trp Asn Arg Glu Gly Gln Thr Val Gln Glu Gly
                165                 170                 175

Ile Lys His Asn Trp Trp Arg Gln Gly Met Met Gly Ser Val Leu Ala
            180                 185                 190

His Tyr Glu Gly Ile Lys Ala Phe Ser Glu Ser Asp Phe Thr Glu Asp
        195                 200                 205

Leu Lys Ser Leu Asp Ile Pro Val Leu Val Leu His Gly Glu Asp Asp
    210                 215                 220

Gln Ile Val Pro Tyr Ala Gln Ala Pro Arg Ala Ala Lys Leu Leu Lys
225                 230                 235                 240

Asn Gly Lys Leu Ile Ser Tyr Pro Gly Phe Ser His Gly Met Pro Thr
                245                 250                 255

Ile Asn Ala Glu Thr Ile Asn Asn Asp Ile Leu Ala Phe Ile Lys
            260                 265                 270